# EUROPEAN PATENT SPECIFICATION

(11) **EP 3 097 162 B1**
(45) Date of publication and mention of the grant of the patent: **23.09.2020**
(21) Application number: 15702920.8
(22) Date of filing: 22.01.2015
(51) Int. Cl.: C09J 133/10, A61L 2/00, A61L 15/58

(54) **GAMMA RESISTANT ADHESIVES**
GAMMARESISTENTE KLEBSTOFFE
ADHÉSIFS RÉSISTANT AUX RAYONS GAMMA

(30) Priority: 22.01.2014 US 201461930017 P
(43) Date of publication of application: 30.11.2016
(73) Proprietor: Adhesives Research, Inc., Pennsylvania 17327 (US)
(72) Inventor: ALLEN, Michael H., York, Pennsylvania 17402 (US); BAER, William C., Harrisburg, Pennsylvania 17110 (US)
(74) Representative: Beck Greener LLP
(86) International application number: PCT/US2015/012352
(87) International publication number: WO 2015/112663

(56) References cited:
- EP-A2- 0 175 562
- WO-A1-2006/018594
- JP-A- 2002 306 589

## Description

### RELATED APPLICATION

### FIELD OF THE INVENTION

The present application is directed toward the field of pressure sensitive adhesive compositions and more particularly to such compositions that exhibit resistance to degradation by gamma radiation.

### BACKGROUND OF THE INVENTION

Adhesive technologies are widely used in wound care and other fields in which the adhesive compositions are subject to sterilization processes, which may be carried out separately on the adhesive or in combination with a bandage or other construct with which the adhesive is employed.

Among the most common form of sterilization is gamma irradiation. However, the polymers used in many conventional wound care adhesives, which are generally based on acrylic or silicone-based polymers, undergo cross-linking when subjected to gamma irradiation. The cross-linking causes the polymer to become more brittle and eliminates its effectiveness as a wound care adhesive. Gamma irradiation also causes conventional wound care adhesives to yellow. This results in both an unpleasant aesthetic as well as a separate loss of functionality in the form of reduced adhesion to the skin.

As a result, ethylene oxide (EtO) sterilization is commonly employed to sterilize wound care adhesives. While effective at avoiding embrittlement and yellowing, EtO sterilization has its own drawbacks, some of which include longer sterilization times and complicated validation processes (i.e. to verify the process was effective). The use of EtO is further undesirable because that compound is carcinogenic and highly explosive.

An adhesive that is capable of sterilization by gamma irradiation, but which exhibits sufficient gamma irradiation resistance that prevents polymer embrittlement and yellowing after sterilization, would be desirable in the art.

### BRIEF DESCRIPTION OF THE INVENTION

The invention is defined by the appending claims. Exemplary embodiments are directed to polymers and adhesives formed from such polymers that exhibit resistance to gamma irradiation and as a result, do not undergo embrittlement or yellowing after gamma sterilization.

According to a first aspect of the invention is provided an adhesive comprising a methacrylate copolymer, wherein the methacrylate copolymer includes:45% to 49% by weight of at least one methacrylate monomer component;45% to 49% by weight of at least one acrylic or silicone monomer component, and2% to 10% by weight acrylic acid or 2-hydroxyethyl acrylate; wherein the adhesive is gamma resistant.

According to another aspect of the invention is provided a medical device includes an adhesive, wherein the adhesive includes a methacrylate copolymer, and wherein the methacrylate copolymer includes at least one methacrylate monomer component and at least one acrylic or silicone monomer component. The adhesive is gamma resistant and is a low trauma adhesive.

Other features and advantages of the present invention will be apparent from the following more detailed description of exemplary embodiments, which illustrate, by way of example, the principles of the invention.

### DETAILED DESCRIPTION OF THE INVENTION

Exemplary embodiments are directed to polymers and more particularly to adhesives such as, for example, those useful for wound care applications. Embodiments of the present disclosure, in comparison to methods and products not utilizing one or more features disclosed herein, exhibit resistance to gamma irradiation and as a result, do not undergo embrittlement or yellowing after gamma sterilization. Accordingly, exemplary embodiments are also directed to providing the gamma resistant adhesives described herein and irradiating those adhesives (and/or any medical device with which they may be employed) with gamma radiation.

As used herein, the reference to "gamma resistant" means the adhesive has less than or equal to a 20% change in peak force and less than or equal to a 15% change in percent loss when comparing the adhesive before and after gamma irradiation of 25 kGy.

Conventional acrylic and silicone adhesives for wound care undergo physical changes due to degradation that impact the adhesive performance after gamma irradiation. Exemplary embodiments resist physical changes during gamma irradiation; this results in an adhesive that exhibits similar loss properties before and after gamma irradiation. The exemplary embodiments are formulated by the use of methacrylate monomers in combination with acrylic or silicone monomers to generate a methacrylate copolymer that is resistant to gamma irradiation. That is, the base polymer of the adhesive composition is itself gamma resistant. Accordingly, a gamma resistant system is achieved that does not require any of the additives that are sometimes used with conventional systems to attempt to offset embrittlement caused by the gamma irradiation, although such additives are not precluded.

The methacrylate copolymers in accordance with exemplary embodiments result in a copolymer that exhibits methacrylate degradation upon gamma irradiation in the form of chain scission. This methacrylate degradation offsets the degradation seen in acrylic polymers and silicone-based systems, which undergo pendant or backbone cross-linking during exposure to gamma irradiation.

Thus, the methacrylate copolymer systems described herein achieve a balance with the competing degradation mechanisms. Chain scission in the methacrylate portions of the copolymer reduces the overall polymer molecular weight while pendant cross-linking in the acrylate and/or silicone portions of the copolymer increases the polymer molecular weight, such that the net effect after gamma sterilization is that the adhesive properties (measured by change in peak force and percent loss) do not exhibit a substantial change.

Acrylic monomers that may be used in accordance with exemplary embodiments of the invention include any acrylic monomers, with a preference for those for which a homopolymer of that monomer has a peak force of less than 250 g. In some embodiments, the acrylic monomer is or includes 2-ethylhexyl acrylate.

Suitable methacrylate copolymers include those obtained from the copolymerization of methacrylic acid alkyl ester monomers which include, by way of example and not of limitation, methyl methacrylate, ethyl methacrylate, propyl methacrylate, butyl methacrylate, hexyl methacrylate, octyl methacrylate, nonyl methacrylate, isononyl methacrylate, isodecyl methacrylate, undecyl methacrylate, lauryl methacrylate, tridecyl methacrylate, stearyl methacrylate, 2-hydroxyethyl methacrylate, methacrylic acid, and 2-ethylhexyl methacrylate.

The copolymers used in exemplary embodiments of the invention are based on at least about 10% by weight, and up to about 95% by weight, of one or more methacrylate monomers, more typically about 25% to about 60%, and in some cases about 50%. Any methacrylate compound may be employed, although methacrylate compounds having eight or more carbon atoms are preferred. Exemplary methacrylate compounds include lauryl methacrylate, tridecyl methacrylate, isodecyl methacrylate, and stearyl methacrylate. It is desireable that the acrylic copolymer exhibits a glass transition temperature below -20 °C to promote adhesion to the skin.

In additional exemplary embodiments, the compositions for the methacrylate copolymer are described by the formulation: about 45% to about 49%, alternatively about 46% to about 48%, by weight acrylic or silicone monomer component, about 45% to about 49%, alternatively about 46% to about 48%, by weight methacrylate monomer component; and about 2% to about 10%, alternatively about 4% to about 8%, by weight acrylic acid or 2-hydroxyethyl acrylate. Suitable combinations of monomer components may include, but are not limited to: 2-ethylhexyl acrylate with lauryl methacrylate and acrylic acid; 2-ethylhexyl acrylate with lauryl methacrylate and 2-hydroxyethyl acrylate; 2-ethylhexyl acrylate with tridecyl methacrylate and acrylic acid; 2-ethylhexyl acrylate with tridecyl methacrylate and 2-hydroxyethyl acrylate; 2-ethylhexyl acrylate with isodecyl methacrylate and acrylic acid; 2-ethylhexyl acrylate with isodecyl methacrylate and 2-hydroxyethyl acrylate; 2-ethylhexyl acrylate with stearyl methacrylate and acrylic acid; and 2-ethylhexyl acrylate with stearyl methacrylate and 2-hydroxyethyl acrylate.

The copolymers may be formed through a solvent reaction mechanism using a suitable common solvent and radical polymerization initiator according to any conventional manner of formulating an acrylic copolymer. Suitable initiators include, but are not limited to, 2,2'-azobis(2-methylbutyronitrile) (available commercially as VAZO® 67 by DuPont) and 1,1'-azobis(cyclohexanecarbonitrile) (available commercially as VAZO® 88 by DuPont).

Exemplary embodiments are further directed to adhesive compositions employing the methacrylate copolymer as a base component and are particularly suitable for low trauma adhesive formulations suitable for wound care applications including incise drapes, securing intravenous sites, and vacuum assisted closure. Thus, the present invention also relates to a wound dressing, affixing tape or other medical device for skin application that includes a substrate coated on at least one side with the adhesives described herein.

By "low trauma" it is meant that the adhesive compositions have a peel strength of less than or equal to 1.64 N/cm on skin and a pain rating of less than 2 on the Wong-Baker pain scale, a scale recognized in the medical field to quantify pain intensity measurement.

The adhesive compositions may be applied directly to a medical dressing or other device, or be formed as a single or double sided tape for use in the later manufacture of a medical dressing or for direct use as an affixing tape to secure the dressing or be formed as a transfer film. A siliconized or other release liner can be applied overlying the adhesive layer to protect it prior to use.

In some embodiments, the dressing to which the adhesive is applied is a thin polymeric film, such as a polyurethane film. These films are flexible and can follow the surface contours of the skin and often have high water vapor transmission rates to allow moisture to escape from the wound area. In still other embodiments, a support layer of paper, plastic or other suitable material may be used to improve handleability of the polymeric film prior to its initial application.

While the adhesive formulations are of a methacrylate copolymer base as described herein, other components may also be present in the final formulation, such as a tackifier and/or plasticizer present along with the methacrylate copolymer. Both the plasticizer and tackifier, if employed, are selected for their biocompatibility and compatibility with the base methacrylate copolymer.

Suitable plasticizers include triisodecyl trimellitate; tributyl trimellitate; tri-n-hexyl trimellitate; tris n-(c7-11l)alkyl ester branched and linear 1,2,4 benzenetricarboxylic acid; butyl benzoate; di-ethylhexylphthalate; di-octylphthalate; di-butylphthalate; diethylhexyl adipate; dibutyl adipate; triethyl citrate; tributyl citrate; acetyl triethyl citrate; acetyl trin-butyl citrate; n-butyryl tri-n-hexyl citrate; triacetin; glycerin; caprylic/capric triglyceride; tricaprin; tricaprylin; propylene glycol dicaprate; propylene glycol dicaprylate/dicaprate; poly(ethylene glycol) (PEG); hydrogenated vegetable oil; hydrogenated seed oil; PEG dilaurate; PEG diethylhexylonate; silicone-based oils; simethicone; and combinations thereof.

Exemplary tackifiers include those selected from the group consisting of rosin esters, polymerized rosins, hydrogenated rosins, polyterpenes, styrenated terpenes, polymerized hydrocarbon resins, alpha methyl styrenes, alpha methyl styrene phenolics and combinations thereof. Exemplary tackifiers include those which are commercially available as a petroleum-based light colored aliphatic hydrocarbon resin (commercially available as ESCOREZ 1310 LC by ExxonMobil), a white aromatic hydrocarbon resin (commercially available as SYLVARES® SA120 by Arizona Chemical), a terpene phenol thermoplastic resin (commercially available as SYLVARES® TP105 by Arizona Chemical), a pale thermoplastic ester of hydrogenated rosin derived from glycerol and a highly stabilized rosin (commercially available as FORAL 85 by Eastman), and a phenol-modified copolymer of styrene and alpha methyl styrene (commercially available as SYLVARES® 540 by Arizona Chemical).

Exemplary embodiments may also employ a cross-linking agent. The cross-linking agent may be present up to about 5% by weight of the base polymer on a solids basis, more typically up to about 3% by weight on a solids basis, and in some cases in the range of about 1.5% to about 2.25% by weight of the methacrylate base copolymer on a solids basis. Any suitable cross-linking agent may be used, including isocyanates, aziridines, metal chelates and combinations thereof, by way of example only.

Other additives in the types and amounts as are known in the art for use in conventional pressure-sensitive adhesives may also be employed, provided those additives do not adversely affect the properties otherwise sought to be achieved. Such additives may include antioxidants, stabilizing agents for enhanced shelf-life, and the like, although one of the advantages of exemplary embodiments is that agents sometimes added to other adhesives to stabilize against gamma sterilization are not necessary and are preferably omitted.

### EXAMPLES

The invention is further described by way of the following examples, which are presented by way of illustration, not of limitation.

### Example 1

To a 50 mL reaction vessel was added 22.0 g ethyl acetate, 36.0 mg VAZO® 67 initiator, and a total of 18.0 g of monomers (49% by weight 2-ethylhexyl acrylate, 49% by weight tridecyl methacrylate, and 2% by weight acrylic acid). The solution was mixed homogenously, nitrogen-purged, and immersed in a 70 °C water bath for 24 h. The resulting product was then cooled to room temperature.

### Reference Example 2

A second example was formulated in the same manner as Example 1, except that the weight percentages that made up the 18.0 g of monomer were 62.5% 2-ethylhexyl acrylate, 27.5% isodecyl methacrylate, and 10% 2-hydroxyethyl acrylate.

### Reference Example 3

A third example was formulated in the same manner as Examples 1 and 2, except that the weight percentages that made up the 18.0 g of monomer were 50% 2-ethylhexyl acrylate and 50% lauryl methacrylate.

### Comparative Example 1

A comparative example was formulated in the same manner as Example 1, except that the weight percentages that made up the 18.0 g monomer were 99.5% 2-ethylhexyl acrylate and 0.5% acrylic acid.

For each of Examples 1-3 and Comparative Example 1, the resulting solutions were coated onto a siliconized polyester liner at a 50 micron thickness and dried at 150 °C for 6 min. The polymers were then laminated and layered onto a 50 micron polyester film to achieve a thickness of 250 microns for compliance testing of each individual polymer.

Peak force change, percent loss, and other compliance measurements are typically performed using a Texture Analyzer, such as a TA-XT2i Texture Analyzer (available from Texture Technologies Corporation) connected to a computer programmed with the texture analyzer software. The method parameters for the test, are outlined below in Table 1, the results of which are referred to herein as the TA Peak Force.

**Table 1**

| **Parameter** | **Definition** | **Specification** |
|---|---|---|
| Mode | The type of test being run | Force/Comp. |
| Option | The type of test sequence being run | Hold Until Time |
| Distance | The set height at which the probe resides prior to testing and after the test is complete | 50.0 mm |
| Pretest Speed | How fast the probe travels at the start of the run until the trigger point is reached | 4.00 mm/sec |
| Trigger Point | The point at which a set minimal force is measured by the load cell as the probe contacts the sample surface. When the trigger force is reached, the test is started. | 1.0g |
| Speed | How fast the probe travels after the trigger point through compression to the designated force | 0.50 mm/sec |
| Time | The amount of time that the probe resides on the sample surface after the force has been achieved | 140.00 sec |
| Post - Speed | The speed at which the probe returns to the starting point where the test is finished | 8.00 mm/sec |
| Probe | The type of ball probe used for testing | TA-8 1/4" |

During sample preparation, liquid adhesive is direct coated onto a siliconized liner and dried. The dried adhesive is then layered onto a 50 micron thick polyester film until a thickness of 280 ± 25 micrometers has been achieved. During the test, the TA-8 probe (¼" ball probe) moves into contact with the surface of the adhesive at a downward rate of 4.00 mm/second until a trigger force of 1 gram is registered on the load cell. The adhesive layer is then compressed to 50% of its total thickness at a rate of 0.50 millimeters/second which triggers data collection for the next 140 seconds. Throughout the entire test, the instrument continuously measures the change in force over time. Data on force versus time is generated into a graph on the computer. The initial peak force is the force required to compress the adhesive layer to 50% of its total thickness. The percent loss is related to the change in force over time (i.e., the change in initial force to the final force over the 140 second period of data collection).

This methodology was used to determine the compliance of the adhesives of Examples 1-3 and Comparative Example 1 both before and after gamma irradiation at 25 kGy. The percent loss measurements are shown below in Table 2 along with the corresponding change after gamma irradiation.

**Table 2. Creep compliance properties of polymers before and after gamma irradiation.**

| **Example** | **Pre-Gamma Peak Force (g)** | **Pre-Gamma % loss (%)** | **Post-Gamma Peak Force (g)** | **Post-Gamma % loss (%)** | **% Change Peak Force** | **% Change % loss** |
|---|---|---|---|---|---|---|
| 1 | 109 | 98.4 | 128 | 92.4 | 17.4 | 6.10 |
| 2 | 149 | 99.1 | 171 | 92.7 | 14.8 | 6.46 |
| 3 | 84.1 | 99.6 | 261 | 101 | 19.6 | 5.02 |
| Comp. 1 | 36.1 | 979.17 | 51.77 | 75.22 | 43.4 | 22.6 |

The following examples 4 to 19 are reference examples.

### Example 4

In a nitrogen purged reaction vessel heated to 90 °C the following mixture was added over 20 min: 60.36 g ethyl acetate, 65.46 g 2-ethylhexyl acrylate, 65.46 g lauryl methacrylate, 8.36 g 2-hydroxyethyl acrylate, and 0.36 g VAZO® 88 initiator.

After stirring at 86 °C for 30 min, the following solution was added to the reaction mixture during the course of 1 h: 69.64 g ethyl acetate, 75.54 g 2-ethylhexyl acrylate, 75.54 g lauryl methacrylate, 9.64 g 2-hydroxyethyl acrylate, and 0.41 g VAZO® 88.

After polymerizing for 1 h at 86 °C, 50.00 g ethyl acetate was added quickly to the reaction and stirred for 90 min. An additional 50.00 g ethyl acetate was added to the polymerization and stirred for 80 min. Then, 75.00 g ethyl acetate and 1.80 g VAZO® 67 was added to the polymerization and the temperature increased to 90 °C for 45 min, upon which the reaction was cooled to room temperature.

To 100 parts (solid content) of the solvent-based methacrylic copolymer the following was blended to form a clear solution: 0.33 parts aromatic polyisocyanate prepolymer based on diphenylmethane diisocyanate cross-linking agent (commercially available as DESMODUR E28 by Bayer MaterialScience), 17.66 parts medium chain caprylic/capric triglycerides (commercially available as MMESTER E7000 by Kraft Chemical Company), 2.99 parts 2,2'-methylenebis(4-methyl-6-tert-butylphenol)monoacrylate (commercially available as IRGANOX® 3052), 1.00 parts pentaerythritol tetrakis(3-(3,5-di-tert-butyl-4-hydroxyphenyl)propionate) (commercially available as IRGANOX® 1010 by BASF Resin), 1.00 parts dilauryl thiodipropionate (commercially available as SONGNOX® DLTDP by Songwon International Ag), and 1.00 parts tris(2,4 -ditertbutylphenyl) phosphite (commercially available as IRGAFOS® 168 by BASF Resins).

The resulting solution was coated onto a siliconized polyester liner at a 50 micron thickness and dried at 150 °C for 6 min. The adhesive was then laminated and layered onto a 50 micron polyester film to achieve a thickness of 250 microns for compliance testing. The sample was cured for 3 days at 65 °C.

TA Peak Force methodology was employed to measure compliance of the adhesive of Example 4, which yielded a peak force of 62.9 g and a percent loss of 83.5%. An additional example (Example 4A) was made in the same manner, except for the omission of IRGAFOS® 168 and SONGNOX® DLTDP; that adhesive exhibited a peak force of 69.9 g and a percent loss of 84.2%.

When the adhesives of Examples 4 and 4A were subjected to approximately 25 kGy gamma irradiation, creep properties were re-evaluated.

The adhesive of Example 4 displayed a peak force of 61.4 g and a percent loss of 80.8%. This was a -2.38% change in peak force and a 3.23% change in % loss after gamma irradiation. For Example 4A, the peak force was 67.4 g with a percent loss of 79.5%. This was a -3.58% change in peak force and a 5.58% change in percent loss.

A 50 micron thick layer of the adhesive of Examples 4 and 4A was also laminated onto a 50 micron polyester film for peel adhesion testing. The sample was applied to a stainless steel substrate for 24 hours to measure the peel force between the adhesive layer and the stainless steel panel. The sample was peeled at a speed of 12 inches/minute in a tensile testing instrument at a 180° angle under ordinary temperature and humidity.

The sample in Example 4 exhibited a peel adhesion to stainless steel of 0.32 N/cm prior to gamma irradiation. After gamma irradiation at 25 kGy, the peel adhesion underwent negligible change to 0.34 N/cm (-6.93% change in peel adhesion). The sample of Example 4A exhibited peel adhesion of 0.66 N/cm prior to gamma irradiation and decreased to 0.47 N/cm post-gamma sterilization (29.4% change in peel adhesion).

### Comparative Example 1A

To 100 parts Comparative Example 1 (solid content) of the solvent-based copolymer the following was blended to form a clear solution: 0.09 parts of trimethylolpropane tris(2-methyl-1-aziridine propionate) cross-linking agent (commercially available as PZ-28), 17.55 parts MMESTER E7000, 2.99 parts IRGANOX® 3052, 1.00 parts IRGANOX® 1010.

### Comparative Example 2

Comparative Example 2 was formulated in the same manner as Example 1, except that the weight percentages that made up the 18.0 g of monomer were 94% 2-ethylhexyl acrylate and 6% 2-hydroxyethyl acrylate. To 100 parts (solid content) of the solvent-based copolymer the following was blended to form a clear solution: 0.28 parts DESMODER E28, 17.16 parts MMESTER E7000, 2.99 parts IRGANOX® 3052, 1.00 parts IRGANOX® 1010.

50 micron thick adhesive layers of Comparative Examples 1A and 2 and Example 4A were laminated onto 50 micron polyester films for peel adhesion testing to skin. The samples were applied to skin for 24 hours to measure the peel force between the adhesive layer and skin. The samples were peeled at a speed of 12 inches/minute in a tensile testing instrument at a 90° angle under ordinary temperature and humidity.

The sample in Example 4A exhibited a peel adhesion to skin of 0.64 N/cm prior to gamma irradiation. After gamma irradiation at 25 kGy, the peel adhesion underwent minimal change to 0.55 N/cm (15.2% change in peel adhesion). Comparative Example 1A exhibited peel adhesion of 1.20 N/cm prior to gamma irradiation and decreased to 0.77 N/cm post-gamma sterilization (35.5% change in peel adhesion). Comparative Example 2 exhibited peel adhesion of 0.75 N/cm prior to gamma irradiation and decreased to 0.57 N/cm post-gamma sterilization (24.7% change in peel adhesion). It can be seen from these examples, incorporating methacrylic monomers into the adhesive reduces the change in peel adhesion to skin after gamma sterilization.

**Table 3**

| **Example No.** | **Monomer (1)** | **Monomer (2)** | **% monomer 1** | **% monomer 2** |
|---|---|---|---|---|
| Comp. 3 | 2-ethylhexyl acrylate | None | 100 | 0 |
| 5 | 2-ethylhexyl acrylate | Lauryl Methacrylate (A) | 75 | 25 |
| 6 | 2-ethylhexyl acrylate | Lauryl Methacrylate (A) | 50 | 50 |
| 7 | 2-ethylhexyl acrylate | Tridecyl Methacrylate | 90 | 10 |
| 8 | 2-ethylhexyl acrylate | Tridecyl Methacrylate | 75 | 25 |
| 9 | 2-ethylhexyl acrylate | Tridecyl Methacrylate | 50 | 50 |
| 10 | 2-ethylhexyl acrylate | Isodecyl Methacrylate | 90 | 10 |
| 11 | 2-ethylhexyl acrylate | Isodecyl Methacrylate | 80 | 20 |
| 12 | 2-ethylhexyl acrylate | Isodecyl Methacrylate | 70 | 30 |
| 13 | 2-ethylhexyl acrylate | Stearyl Methacrylate | 75 | 25 |
| 14 | 2-ethylhexyl acrylate | Stearyl Methacrylate | 50 | 50 |
| 15 | Stearyl Methacrylate | None | 100 | 0 |
| 16 | Isodecyl Methacrylate | None | 100 | 0 |
| 17 | Tridecyl Methacrylate | None | 100 | 0 |
| 18 | Lauryl Methacrylate | None | 100 | 0 |

### Examples 5-19; Comparative Ex. 3

Additional examples were formulated as shown in Table 3, including a Comparative Example 3 using pure 2-ethylhexyl acrylate for purposes of comparison. All polymerization reactions were run in mini-reactors at 45% solids in ethyl acetate, 0.2 wt. % VAZO 67, in a 70 °C water bath overnight. Stearyl methacrylate homopolymerization was performed in a 50/50 (w/w) heptane/ethyl acetate mixture. NP101 was a polyester pressure sensitive adhesive purchased from Nippon Gohsei.

All polymers were prepared using creep procedures (layer samples to achieve 10-12 mil thickness). The formed samples were subjected to approximately 25 kGy of gamma irradiation. Compliance measurements were obtained before and after irradiation; results are shown in Table 4.

**Table 4**

| **Ex. No.** | **Creep Pre-γ Peak Force** | **Creep Pre-γ % Loss** | **Creep Post-γ Peak Force** | **Creep Post-γ % Loss** | **% Change Peak Force** | **% Change % Loss** |
|---|---|---|---|---|---|---|
| Comp 3 | 64.9 | 100.1 | 131.8 | 95.3 | 103.1 | 4.80 |
| 5 | 62.6 | 100 | 140 | 96.4 | 123.6 | 3.60 |
| 6 | 84.1 | 99.6 | 100.6 | 94.6 | 19.6 | 5.02 |
| 7 | 64.2 | 100 | 149.3 | 97.0 | 132.6 | 3.00 |
| 8 | 58.7 | 100.3 | 130.9 | 97.1 | 123.0 | 3.19 |
| 9 | 79.9 | 99.8 | 98.2 | 95.2 | 22.9 | 4.61 |
| 10 | 71.3 | 100.1 | 159.9 | 98.0 | 124.3 | 2.10 |
| 11 | 78.5 | 100 | 131.7 | 98.9 | 67.8 | 1.10 |
| 12 | 87.6 | 100 | 127.1 | 98.3 | 45.1 | 1.70 |
| 13 | 48.4 | 100.3 | 155.5 | 97.9 | 221.3 | 2.39 |
| 14 | 45.5 | 100 | 146.9 | 99.4 | 222.9 | 0.60 |
| 15 | 1214.6 | 5.3 | 1286.3 | 7.7 | 5.9 | -45.28 |
| 16 | 459.7 | 97.5 | 438.8 | 98 | -4.5 | -0.51 |
| 17 | 184.8 | 99.3 | 224.8 | 93.6 | 21.6 | 5.74 |
| 18 | 209.6 | 98.6 | 221.3 | 94 | 5.6 | 4.67 |

### Examples 20-25

To a 50 mL reaction vessel was added 22.0 g ethyl acetate, 36.0 mg VAZO® 67 initiator, and a total of 18.0 g of monomers (45% tridecyl methacrylate, 45% 2-ethylhexyl acrylate, and 10% 2-hydroxyethyl acrylate). The solution was mixed homogenously, nitrogen-purged, and immersed in a 70 °C water bath for 24 h. The resulting product was then cooled to room temperature.

To a 50 mL reaction vessel was added 22.0 g ethyl acetate, 36.0 mg VAZO® 67 initiator, and a total of 18.0 g of monomers (47.5% lauryl methacrylate, 47.5% 2-ethylhexyl acrylate, 3% acrylamide, and 2% acrylic acid). The solution was mixed homogenously, nitrogen-purged, and immersed in a 70 °C water bath for 24 h. The resulting product was then cooled to room temperature.

To a 50 mL reaction vessel was added 22.0 g ethyl acetate, 36.0 mg VAZO® 67 initiator, and a total of 18.0 g of monomers (40% lauryl methacrylate, 55% 2-ethylhexyl acrylate, and 5% 2-hydroxyethyl acrylate). The solution was mixed homogenously, nitrogen-purged, and immersed in a 70 °C water bath for 24 h. The resulting product was then cooled to room temperature.

To a 50 mL reaction vessel was added 22.0 g ethyl acetate, 36.0 mg VAZO® 67 initiator, and a total of 18.0 g of monomers (70% isodecyl methacrylate, 27% 2-ethylhexyl acrylate, and 3% acrylic acid). The solution was mixed homogenously, nitrogen-purged, and immersed in a 70 °C water bath for 24 h. The resulting product was then cooled to room temperature.

To a 50 mL reaction vessel was added 22.0 g ethyl acetate, 36.0 mg VAZO® 67 initiator, and a total of 18.0 g of monomers (70% lauryl methacrylate, 25% methyl methacrylate, and 5% 2-hydroxyethyl acrylate). The solution was mixed homogenously, nitrogen-purged, and immersed in a 70 °C water bath for 24 h. The resulting product was then cooled to room temperature.

To a 50 mL reaction vessel was added 22.0 g ethyl acetate, 36.0 mg VAZO® 67 initiator, and a total of 18.0 g of monomers (97% lauryl methacrylate, 3% acrylic acid). The solution was mixed homogenously, nitrogen-purged, and immersed in a 70 °C water bath for 24 h. The resulting product was then cooled to room temperature.

## Claims

1. An adhesive comprising a methacrylate copolymer, wherein the methacrylate copolymer includes:
45% to 49% by weight of at least one methacrylate monomer component;
45% to 49% by weight of at least one acrylic or silicone monomer component, and
2% to 10% by weight acrylic acid or 2-hydroxyethyl acrylate;
wherein the adhesive is gamma resistant.

2. The adhesive of Claim 1, wherein the adhesive is a low trauma adhesive.

3. The adhesive of Claim 1, wherein the at least one methacrylate monomer component is a methacrylic acid alkyl ester monomer.

4. The adhesive of Claim 1, wherein the at least one methacrylate monomer component is selected from the group consisting of hexyl methacrylate, octyl methacrylate, nonyl methacrylate, isononyl methacrylate, isodecyl methacrylate, undecyl methacrylate, lauryl methacrylate, tridecyl methacrylate, stearyl methacrylate, 2-hydroxyethyl methacrylate, 2-ethylhexyl methacrylate, and combinations thereof.

5. The adhesive of Claim 4, wherein the at least one methacrylate monomer component is selected from the group consisting of lauryl methacrylate, tridecyl methacrylate, isodecyl methacrylate, stearyl methacrylate, and combinations thereof.

6. The adhesive of Claim 1, wherein the at least one acrylic or silicone monomer component is an acrylic monomer for which its homopolymer has a peak force of less than 250 g as determined by the method disclosed in the description.

7. The adhesive of Claim 1, wherein the at least one acrylic monomer component is 2-ethylhexyl acrylate.

8. The adhesive of Claim 1, wherein the adhesive is a pressure-sensitive adhesive.

9. The adhesive of Claim 1, wherein the adhesive further comprises a plasticizer.

10. The adhesive of Claim 1, wherein the adhesive further comprises a tackifier.

11. The adhesive of Claim 1, wherein the adhesive further comprises a cross-linking agent.

12. The adhesive of Claim 1, wherein the adhesive further comprises an antioxidant.

13. An adhesive according to any of Claims 1 to 12, wherein the methacrylate copolymer further includes:
at least one additive selected from the group consisting of a plasticizer, a tackifier,
a cross-linking agent, an antioxidant and combinations thereof,
wherein the adhesive is a pressure-sensitive adhesive, and
wherein the adhesive is a low trauma adhesive.

14. A medical device comprising an adhesive according to any of Claims 1 to 13, wherein the medical device is optionally selected from the group consisting of incise drapes, affixing tape, single sided tape, double sided tape and transfer film.

## Patentansprüche

1. Klebstoff, umfassend ein Methacrylatcopolymer, wobei das Methacrylatcopolymer Folgendes umfasst:
45 bis 49 Gew.-% mindestens einer Methacrylatmonomerkomponente;
45 bis 49 Gew.-% mindestens einer Acryl- oder Silikonmonomerkomponente und
2 bis 10 Gew.-% Acrylsäure oder 2-Hydroxyethylacrylat;
wobei der Klebstoff gammabeständig ist.

2. Klebstoff nach Anspruch 1, wobei der Klebstoff ein geringes Trauma verursachender Klebstoff ist.

3. Klebstoff nach Anspruch 1, wobei die mindestens eine Methacrylatmonomerkomponente ein Methacrylsäurealkylestermonomer ist.

4. Klebstoff nach Anspruch 1, wobei die mindestens eine Methacrylatmonomerkomponente ausgewählt ist aus der Gruppe bestehend aus Hexylmethacrylat, Octylmethacrylat, Nonylmethacrylat, Isononylmethacrylat, Isodecylmethacrylat, Undecylmethacrylat, Laurylmethacrylat, Tridecylmethacrylat, Stearylmethacrylat 2-Hydroxyethylmethacrylat, 2-Ethylhexylmethacrylat und Kombinationen davon.

5. Klebstoff nach Anspruch 4, wobei die mindestens eine Methacrylatmonomerkomponente ausgewählt ist aus der Gruppe bestehend aus Laurylmethacrylat, Tridecylmethacrylat, Isodecylmethacrylat, Stearylmethacrylat und Kombinationen davon.

6. Klebstoff nach Anspruch 1, wobei die mindestens eine Acryl- oder Silikonmonomerkomponente ein Acrylmonomer ist, für das sein Homopolymer eine Spitzenkraft von weniger als 250 g aufweist, wie durch das in der Beschreibung offenbarte Verfahren bestimmt wird.

7. Klebstoff nach Anspruch 1, wobei die mindestens eine Acrylmonomerkomponente 2-Ethylhexylacrylat ist.

8. Klebstoff nach Anspruch 1, wobei der Klebstoff ein Haftklebstoff ist.

9. Klebstoff nach Anspruch 1, wobei der Klebstoff ferner einen Weichmacher umfasst.

10. Klebstoff nach Anspruch 1, wobei der Klebstoff ferner einen Klebrigmacher umfasst.

11. Klebstoff nach Anspruch 1, wobei der Klebstoff ferner ein Vernetzungsmittel umfasst.

12. Klebstoff nach Anspruch 1, wobei der Klebstoff ferner ein Antioxidationsmittel umfasst.

13. Klebstoff nach einem der Ansprüche 1 bis 12, wobei das Methacrylatcopolymer ferner Folgendes umfasst:
mindestens ein Additiv ausgewählt aus der Gruppe bestehend aus einem Weichmacher, einem Klebrigmacher, einem Vernetzungsmittel, einem Antioxidationsmittel und Kombinationen davon,
wobei der Klebstoff ein Haftklebstoff ist, und
wobei der Klebstoff ein geringes Trauma verursachender Klebstoff ist.

14. Medizinische Vorrichtung, umfassend einen Klebstoff nach einem der Ansprüche 1 bis 13, wobei die medizinische Vorrichtung gegebenenfalls ausgewählt ist aus der Gruppe bestehend aus Inzisionsfolien, Klebeband, einseitigem Klebeband, doppelseitigem Klebeband und Transferfolie.

## Revendications

1. Adhésif comprenant un copolymère de méthacrylate, dans lequel le copolymère de méthacrylate comporte :
45 % à 49 % en poids d'au moins un composant monomère de méthacrylate ;
45 % à 49 % en poids d'au moins un composant monomère acrylique ou de silicone, et
2 % à 10 % en poids d'acide acrylique ou d'acrylate de 2-hydroxyéthyle ;
dans lequel l'adhésif est résistant aux rayons gamma.

2. Adhésif selon la revendication 1, dans lequel l'adhésif est un adhésif à faible traumatisme.

3. Adhésif selon la revendication 1, dans lequel l'au moins un composant monomère de méthacrylate est un monomère ester alkylique d'acide méthacrylique.

4. Adhésif selon la revendication 1, dans lequel l'au moins un composant monomère de méthacrylate est choisi dans le groupe constitué par le méthacrylate d'hexyle, le méthacrylate d'octyle, le méthacrylate de nonyle, le méthacrylate d'isononyle, le méthacrylate d'isodécyle, le méthacrylate d'undécyle, le méthacrylate de lauryle, le méthacrylate de tridécyle, le méthacrylate de stéaryle, le méthacrylate de 2-hydroxyéthyle, le méthacrylate de 2-éthylhexyle et leurs combinaisons.

5. Adhésif selon la revendication 4, dans lequel l'au moins un composant monomère de méthacrylate est choisi dans le groupe constitué par le méthacrylate de lauryle, le méthacrylate de tridécyle, le méthacrylate d'isodécyle, le méthacrylate de stéaryle et leurs combinaisons.

6. Adhésif selon la revendication 1, dans lequel l'au moins un composant monomère acrylique ou de silicone est un monomère acrylique pour lequel son homopolymère a une force maximale inférieure à 250 g telle que déterminée par le procédé décrit dans la description.

7. Adhésif selon la revendication 1, dans lequel l'au moins un composant monomère acrylique est l'acrylate de 2-éthylhexyle.

8. Adhésif selon la revendication 1, dans lequel l'adhésif est un adhésif sensible à la pression.

9. Adhésif selon la revendication 1, dans lequel l'adhésif comprend en outre un plastifiant.

10. Adhésif selon la revendication 1, dans lequel l'adhésif comprend en outre un agent collant.

11. Adhésif selon la revendication 1, dans lequel l'adhésif comprend en outre un agent de réticulation.

12. Adhésif selon la revendication 1, dans lequel l'adhésif comprend en outre un antioxydant.

13. Adhésif selon l'une quelconque des revendications 1 à 12, dans lequel le copolymère de méthacrylate comporte en outre :
au moins un additif choisi dans le groupe constitué par un plastifiant, un agent collant, un agent de réticulation, un antioxydant et leurs combinaisons,
dans lequel l'adhésif est un adhésif sensible à la pression, et dans lequel l'adhésif est un adhésif à faible traumatisme.

14. Dispositif médical comprenant un adhésif selon l'une quelconque des revendications 1 à 13, dans lequel le dispositif médical est éventuellement choisi dans le groupe constitué par les champs stériles pour incision, un ruban de fixation, un ruban simple face, un ruban double face et un film de transfert.
